# EUROPEAN PATENT APPLICATION

(11) **EP 3 246 313 A1**
(43) Date of publication of application: **22.11.2017**
(21) Application number: 16170429.1
(22) Date of filing: 19.05.2016
(51) Int. Cl.: C07D 213/32, C07D 213/50, C07C 221/00, C07C 225/14

(54) **PROCESS FOR THE MANUFACTURE OF 2-SUBSTITUTED-5-(1-METHYLTHIO)ALKYLPYRIDINES**

(71) Applicant: SOLVAY SA, 1120 Bruxelles (BE)
(72) Inventor: BRAUN, Max Josef, 30900 Wedemark (DE)
(74) Representative: Mross, Stefan P.M.

(57) **Abstract**

Process for the manufacture of 2-substituted-5-(1-methylthio)alkylpyridines and a process for the manufacture of agriculturally or pharmaceutically active substances comprising the process for the manufacture of 2-substituted-5-(1-methylthio)alkylpyridines.

## Description

Process for the manufacture of 2-substituted-5-(1-methylthio)alkylpyridines and a process for the manufacture of agriculturally or pharmaceutically active substances comprising the process for the manufacture of 2-substituted-5-(1-methylthio)alkylpyridines.

2-substituted-5-(1-methylthio)alkylpyridines are important intermediates for the manufacture of agriculturally or pharmaceutically active substances, for example sulfoximine, in particular sulfoxaflor, which are nicotinic acetylcholine receptor competitive modulators. There is a continuing need for the development of improved processes for the manufacture of precursors of such active compounds. US8129539B2 discloses a process for the manufacture of 2-substituted-5-(1-methylthio)alkylpyridines, wherein a sulfur containing enamine is cyclized to form the pyridine. Early introduction of sulfur into process precursors is often associated with instability, complex workup and emission challenges.

The invention thus concerns a process for the manufacture of 5-acetyl-2-substituted-pyridines which comprises one of the steps or reaction steps selected from the group consisting of steps a) and b):
a) reacting a compound of formula (I) with a compound of formula (II) to obtain the compound of formula (III) 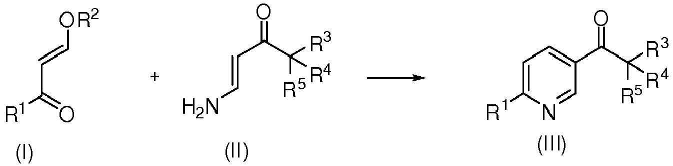 wherein R¹represents C₁-C₄ alkyl or C₁-C₄ haloalkyl, R² represents C₁-C₄-alkyl, R³, R⁴ and R⁵ independently from each other are selected from the group consisting of H and C₁-C₄-alkyl, wherein C₁-C₄-alkyl is optionally substituted.
b) wherein a compound of formula (VI) is reacted with at least one reducing agent to obtain a compound of formula (III) 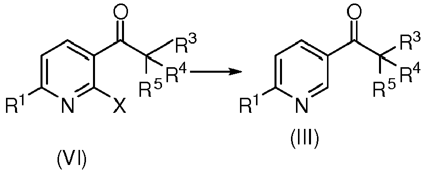 wherein X is selected from the group consisting of F, Cl, Br and I, wherein Br and Cl are preferred, and R¹, R³, R⁴ and R⁵ are as defined above.
   The process for the manufacture of a compound of formula (III) can also comprise a step c) wherein compound of formula (V) is reacted with a halogenation agent to obtain (VI) 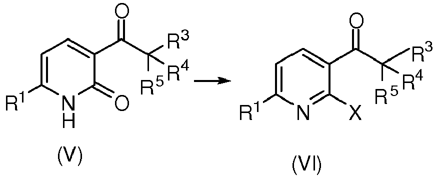 The invention concerns further a process as outlined above to obtain (III), which further comprises a step or reaction step selected from the group consisting of d), e) and f):
d) reacting a compound of formula (VII) with a compound of formula (VIII) to obtain the compound of formula (IX), which is subsequently reacted with NH₃ or NH₄R⁷, wherein R⁷ is selected from the group consisting of OH, Br and Cl to obtain the compound of formula (II) 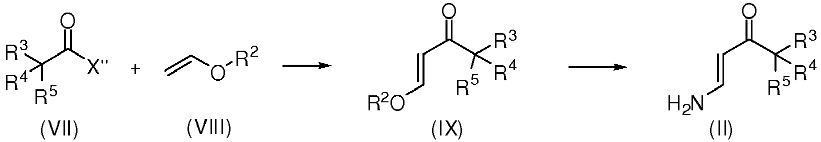
e)reacting a compound of formula (X) with a compound NH₃ or NH₄R⁷, wherein R⁷ is selected from the group consisting of OH, Br and Cl to obtain the compound of formula (II), wherein M is an alkali metal cation, preferably sodium 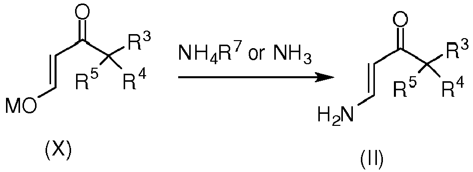
f) reacting a compound of formula (XI) with compound NH₃ or NH₄R⁷, wherein R⁷ is selected from the group consisting of OH, Br and Cl to obtain the compound of formula (II)

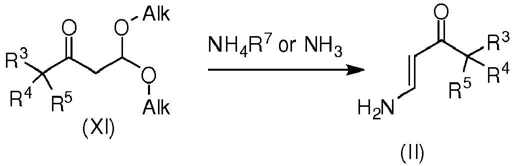

Compounds of formula (XI) can be obtained commercially or made according to synthesis methods know to the skilled artisan. Alk in (XI) denotes lower alkyl, in particular methyl, ethyl, i-propyl, n-propyl, n-butyl, tert-butyl and sec-butyl. Both alkyl groups in (XI) can be selected from the foregoing independently from another.

The invention also concerns a process for the manufacture of a compound according to formula (XII), 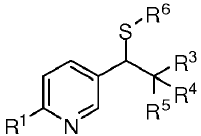 which comprises the process comprising step or step a) or b), and wherein R⁶ is selected from the group consisting of H and C₁-C₄-alkyl, wherein C₁-C₄-alkyl is optionally substituted, and R¹, R³, R⁴, R⁵ and R⁶ are as defined above. This process can further comprise at least one of the steps or steps selected from the group consisting of g), h) or i)
g) Reduction of compound (III) to obtain compound (XIII), halogenation of compound (XIII) to obtain compound (XIV), wherein X' is selected from Br, Cl and I, and thioalkylation of compound (XIV) to obtain compound (XII) 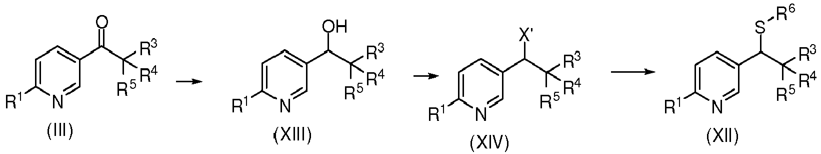
h) Halogenation of (III) with subsequent dehydratization, when at least R³ is H to obtain (XV); thioalkylation to obtain (XVI) and subsequent reduction to obtain (XII), wherein X" is selected from Br and Cl 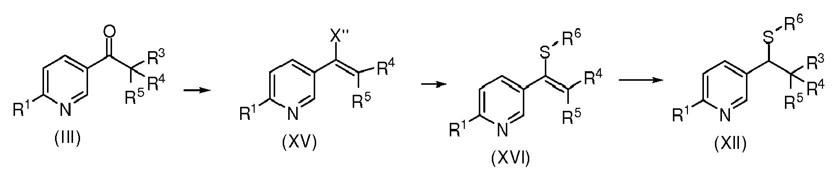
i) Thiation of (III) to obtain (XVII); reduction to obtain (XVIII) and subsequent alkylation to obtain (XII)

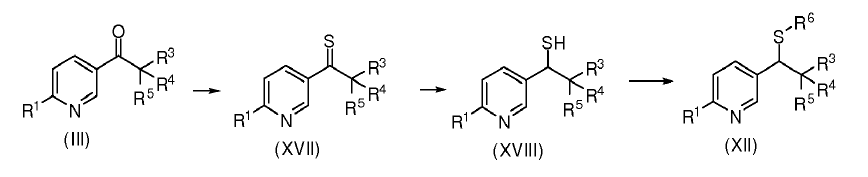

The invention also concerns a process for the manufacture of an agriculturally or pharmaceutically active compound or an intermediary thereof, comprising the process as described before for the manufacture of (III) or (XII).

In the description, formulae including a stereocenter are intended to denote the racemate, (R)-enantiomer, (S)-enantiomer or any non-racemic mixture of the (R)- and (S)-enantiomer. The term "comprising" intends to denote also "consists of". Any singular terms (e.g. "solvent") intend to denote the plural, as well (e.g. "solvents").

In the present invention, the term C₁-C₄ alkyl represents a group selected from methyl, ethyl, i-propyl, n-propyl, n-butyl, tert-butyl and sec.-butyl. The term C₁-C₄ haloalkyl represents a group selected from methyl, ethyl, i-propyl, n-propyl, n-butyl, tert-butyl and sec.-butyl, wherein the group is substituted by at least one halogen. The at least one halogen is selected from the group consisting of F, Br, Cl and I. When haloalkyl group is substituted with more than one halogen atom, the two more halogen atoms can be the same or selected independently.

In the present invention, the term "C₁-C₄-alkyl which is optionally substituted" generally intends to denote a group selected from methyl, ethyl, i-propyl, n-propyl, n-butyl, tert-butyl and sec.-butyl, each of which is optionally substituted by at least one substituent selected from the group consisting of -R', - X', -OR', -SR', -NR'₂, -SiR'₃, -COOR", -CN and -CONR'₂, where R' is hydrogen or a C₁-C₁₂-alkyl group which are the same or different in - CONR'₂, and X' is F, Cl, Br, or I. For the purpose of the present invention, the definition C₁-C₁₂-alkyl comprises the largest range defined herein for an alkyl group. Specifically, this definition comprises, for example, the meanings methyl, ethyl, n-propyl, isopropyl, n-, iso-, sec- and t-butyl, n-pentyl, n-hexyl, 1,3-dimethylbutyl, 3,3-dimethylbutyl, n-heptyl, n-nonyl, n-decyl, n-undecyl and n-dodecyl. Often, methyl, ethyl, n-propyl, isopropyl, n-, iso-, sec- and t-butyl are most preferred residues selected from the group C₁-C₁₂-alkyl.

The invention concerns a process for the manufacture of a 5-acetyl-2-substituted-pyridine, comprising step a), wherein a compound of formula (I) is reacted with a compound of formula (II) to obtain the compound of formula (III):

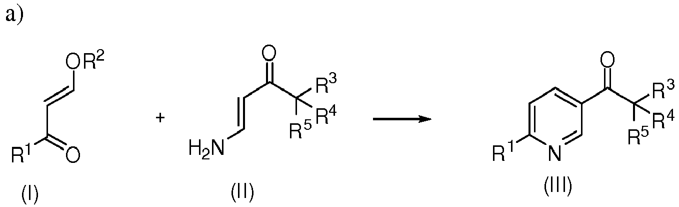

According to the present invention, R¹ represents C₁-C₄ alkyl or C₁-C₄ haloalkyl, R² represents C₁-C₄-alkyl, wherein C₁-C₄-alkyl is optionally substituted, and R³, R⁴ and R⁵ independently from each other are selected from the group consisting of H and C₁-C₄-alkyl, wherein C₁-C₄-alkyl is optionally substituted. Preferably, R¹ is a C₁-C₄ haloalkyl group. When R¹ is a haloalkyl, R¹ preferably is selected from the group consisting of CHF₂, CF₃ and CClF₂. CF₃ is most preferred when R¹ is a haloalkyl group. R² often is a methyl, ethyl, i-propyl or n-propyl group, wherein methyl and ethyl are preferred. Often, at least one of the groups R³, R⁴ and R⁵ is H. In one preferred aspect, R³, R⁴ and R⁵ are H.

Often, step a) is carried out in the presence of a base, which can be an organic or inorganic base. The base can, for example, be selected from the group consisting of metal carbonates, such as sodium carbonate, or from the group consisting of amines, for example NH₃, Et₃N and (i-Pr)₂NH.

The invention further concerns a process for the manufacture of a 5-acetyl-2-substituted-pyridine, comprising step b), wherein a compound of formula (VI) is reacted with at least one reducing agent to obtain a compound of formula (III)

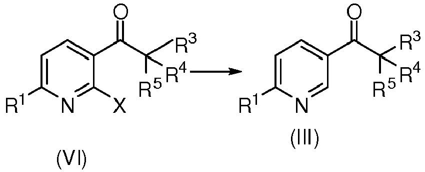

Step b) generally is carried out in the presence of a suitable hydrogenation agent or hydrogen in the presence of a suitable catalyst. H₂ and suitable catalysts, for example Pd/C, Pd or another suitable metal based hydrogenation catalyst, optionally Pd/C, Pd or metal hydrogenation catalysts supported on suitable carriers such as Al₂O₃, is preferred.

In the present invention, the process for the manufacture of a compound of formula (III) can comprise a step c), wherein a compound of formula (V) is reacted with a halogenation agent to obtain (VI)

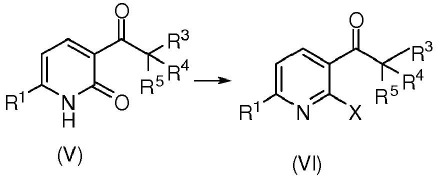

Step c), concerning the reaction of the compound of formula (V) to obtain the compound of formula (VI) generally is carried out in the presence of a suitable halogenation agent. The halogenation agent can, for example, be selected from Cl₂, POCl₃, SO₂Cl₂ or PCl₅ or the corresponding bromination agent. Compounds of formula (V) can be obtained, for example, by the procedure described by R.W. Lang et. al., Helv. Chim. Acta 1998, Vol. 71, p. 596-601, or N. Zanatta et. al., Synthesis 1999, No. 5, p. 765-768.

The compound of formula (III) can also be obtained by the following reaction sequence j) disclosed by J.-N. Desrosiers et al, Org. Lett. 2014, 16, 1724-1727.

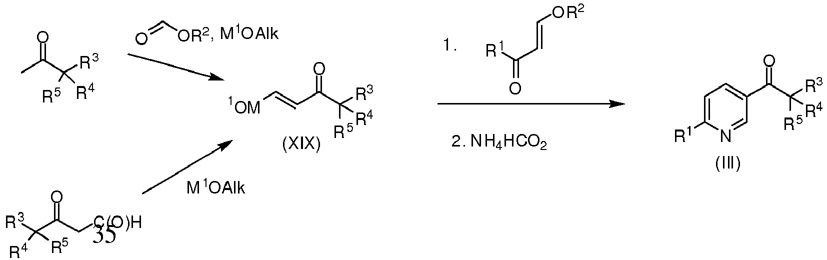

In sequence j), M¹ is an alkali metal selected from Na, K, Li and Cs, wherein K and Na are preferred. Alk is a group selected from methyl, ethyl, n-propyl and i-propyl, preferably Alk is methyl. R¹ to R⁵ are as described above. Sequence j) is carried out preferably in the presence of an aprotic, dipolar solvent, for example dimethylsulfoxide or dimethylformamide. Acetonitrile can also be a suitable solvent. In the step of reacting R¹C(O)CH=CH-OR² with (XIX), temperatures of from 0°C to 80°C, in particular of about 20°C often are suitable. When HCO₂NH₄ is added, temperatures of from 20°C to 100°C, in particular of 70°C can be suitable.

In one aspect of the present invention, the process to obtain (III) which comprises step a) further comprises step d) of reacting a compound of formula (VII) with a compound of formula (VIII) to obtain the compound of formula (IX), which is subsequently reacted with NH₃ or NH₄R⁷, wherein R⁷ is selected from the group consisting of OH, Br and Cl to obtain the compound of formula (II).

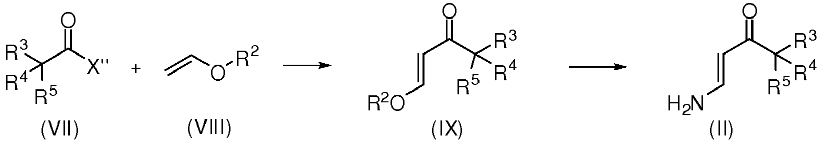

In step d), X" is selected from the group consisting of F, Br and Cl, wherein Br and Cl are preferred. R¹ to R⁵ are defined as above. The reaction of (VII) and (VIII) to obtain (IX) can be carried out in an inert solvent or without additional solvent. Optionally, an acid scavenger can be present. Suitable reaction conditions are described, for example, in EP2310350 and EP1644306. The reaction of (IX) with NH₃ or NH₄R⁷, wherein R⁷ is selected from the group consisting of OH, Br and Cl, wherein NH₃ and NH₄OH are preferred, to obtain the compound of formula (II) often can suitably be effected in the presence of a polar aprotic solvent such as dimethylsulfoxide or dimethylformamide. In step d), R² to R⁵ are described as above. In some aspects, it is of advantage to carry out the reaction of (VII) with (VIII) in the presence of a Lewis acid catalyst, such as AlCl₃ or lanthanide triflates, or a suitable base.

In one aspect of the present invention, the process to obtain (III) which comprises step a) further comprises step e) of reacting a compound of formula (X) with a compound NH₃ or NH₄R⁷, wherein R⁷ is selected from the group consisting of OH, Br and Cl to obtain the compound of formula (II), wherein M is an alkali metal cation, preferably sodium

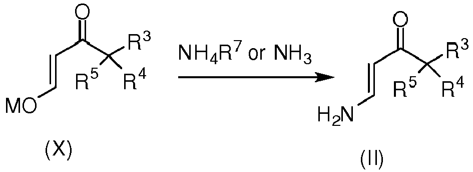

The reaction to obtain (II) often can suitably be carried out in a solvent selected from ethers, such as THF or diisopropylether, or DMSO (dimethylsulfoxide).

Compounds of formula (X) can be obtained, for example, by reaction of a formic acid ester with a carbonyl compound in the presence of an alcoholate, for example reaction of acetone with ethyl formate in the presence of sodium methylate to yield (X) wherein M=Na and R³, R⁴ and R⁵ are H. In one aspect of the present invention, the process to obtain (III) which comprises step a) further comprises step f) of reacting a compound of formula (XI) with compound NH₃ or NH₄R⁷, wherein R⁷ is selected from the group consisting of OH, Br and Cl to obtain the compound of formula (II)

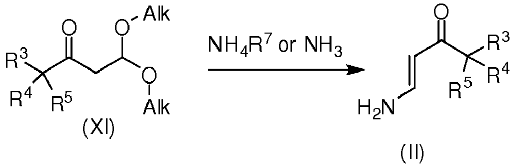

The invention also concerns a process for the manufacture of a compound according to formula (XII), 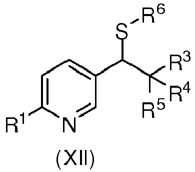 wherein a compound of formula (III) is used as a starting material to obtain (XII) in one or more chemical steps. In particular, such a process to manufacture (XII) from (III) comprises the process for manufacturing (III) as described above. Generally, R⁶ is selected from the group consisting of H and C₁-C₄-alkyl, wherein C₁-C₄-alkyl is optionally substituted. In a preferred aspect, R⁶ is methyl.

In one aspect, the process for the manufacture of a compound according to formula (XII) comprises a step g), wherein a compound of formula (III) is reduced to obtain compound (XIII), followed by halogenation of compound (XIII) to obtain compound (XIV), wherein X' is selected from Br, Cl and I, and thioalkylation of compound (XIV) to obtain compound (XII)

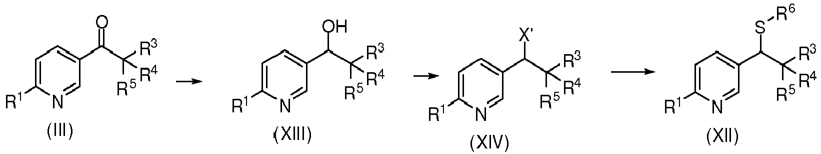

Generally, the reduction of compound (III) is performed with a suitable reducing agent, such as NaBH₄ or H₂ in the presence of a suitable catalyst. When NaBH₄ is selected as reducing agent, the reaction generally is performed in an alcoholic solvent, for example MeOH. When H₂ is used as reducing agent, generally a suitable catalyst is present, for example Pd/C, Pd or another suitable metal based hydrogenation catalyst, or optionally Pd/C, Pd or metal hydrogenation catalysts supported on suitable carriers such as Al₂O₃. The halogenation of (XIII) to obtain (XIV) is performed with suitable halogenation agents, for example selected from COX'₂, PX'₃, POX'₃ or PX'₅, wherein X' is selected from the group consisting of F, Cl and Br, wherein Cl and Br, and in particular Cl, is preferred. The subsequent step of thioalkylation of (XIV) generally is performed with M¹SR⁶, wherein M¹ and R⁶ are described as above. M¹ = Na and R⁶ are particularly preferred.

In another aspect, the process for the manufacture of a compound according to formula (XII) comprises a step h), wherein the compound of formula (III) is halogenated with subsequent dehydratization, when at least R³ is H, to obtain the compound of formula (XV), followed by thioalkylation to obtain (XVI) and subsequent reduction to obtain (XII), wherein X" is selected from Br and Cl. 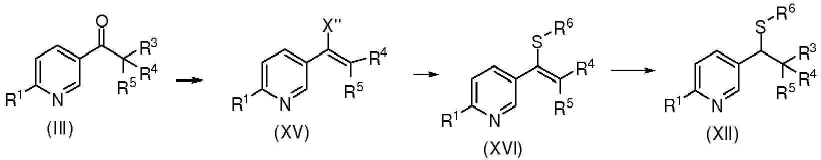 In this step, R³ is H, and R¹, R⁴ and R⁵ are described as above. The halogenation with subsequent dehydratization of (III) is effected in the presence of a suitable agent, for example POX" ₃ or PX"₅, wherein X" is selected from Br and Cl. (XV) is subsequently subjected to a thioalkylation step with M¹SR⁶, wherein M¹ and R⁶ are described as above. The reaction often benefits from acidic reaction medium. The subsequent reduction step of (XVI) to obtain (XII) generally is performed in the presence of H₂ as reducing agent, and a suitable catalyst, for example Pd/C, Pd or another suitable metal based hydrogenation catalyst, or optionally Pd/C, Pd or metal hydrogenation catalysts supported on suitable carriers such as Al₂O₃.

In yet another aspect, the process for the manufacture of a compound according to formula (XII) comprises a step i), which comprises thiation of (III) to obtain (XVII), subsequent reduction to obtain (XVIII) and subsequent alkylation to obtain (XII).

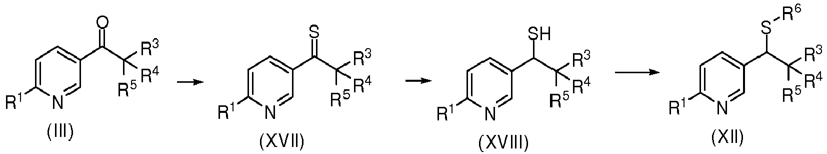

Thiation of the compound of formula (III) can be achieved by reaction with suitable agents, such as P₄S₁₀ or Lawson's reagent. The subsequent reduction of (XVII) to obtain (XVIII) usually is performed by reaction with NaBH₄ or H₂ in the presence of a suitable catalyst, such as metal hydrogenation catalysts or metal hydrogenation catalysts on suitable carriers. Alkylation of (XVIII) to obtain (XII) is generally performed by suitable alkylation agents, such as, for example with MeI, MeBr, MeCl, dimethylsulfoxide, diazomethane or methylmethanesulfonate.

The invention also concerns a process for the manufacture of an agriculturally or pharmaceutically active compound or an intermediary thereof, which comprises the process to obtain (III) as described above, and/or the process to obtain (XII). In one particular aspect, the agriculturally active compound or intermediate is a sulfoximine, in particular sulfoxaflor (CAS No. 946578-00-3).

The compound of formula (XII) obtained by the processes comprising one or more of the steps outlined above can be used for the manufacture of an agriculturally or pharmaceutically active compound or an intermediary thereof, preferably wherein the agriculturally active compound is a sulfoximine, in particular sulfoxaflor.

## Claims

1. Process for the manufacture of a 5-acetyl-2-substituted-pyridines, comprising one of the steps or reaction steps selected from the group consisting of steps a) and b):
a) reacting a compound of formula (I) with a compound of formula (II) to obtain the compound of formula (III) 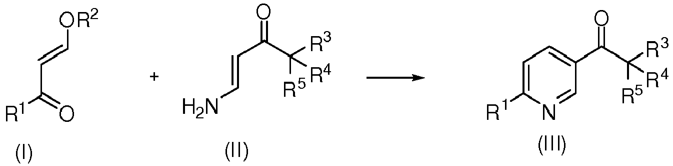 wherein R¹ represents C₁-C₄ alkyl or C₁-C₄ haloalkyl, R² represents C₁-C₄-alkyl, R³, R⁴ and R⁵ independently from each other are selected from the group consisting of H and C₁-C₄-alkyl, wherein C₁-C₄-alkyl is optionally substituted.
b) wherein a compound of formula (VI) is reacted with at least one reducing agent to obtain a compound of formula (III) 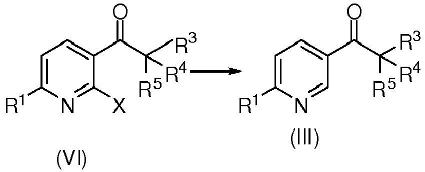 wherein X is selected from the group consisting of F, Cl, Br and I, wherein Br and Cl are preferred, and R¹, R³, R⁴ and R⁵ are as defined above.

2. Process according to claim 1, wherein the process comprises a step c) of reacting a compound of formula (V) with a halogenation agent to obtain (VI), 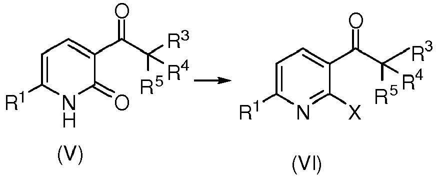 wherein R¹, R³, R⁴, R⁵ and X are defined as above.

3. Process according to claim 1 or 2, wherein R² is selected from the group consisting of CH₃, C₂H₅, CH₂CH₂CH₃ and CH(CH₃)₂.

4. Process according to anyone of claims 1 to 3, wherein R¹ is selected from the group consisting of CF₃, CF₂H, CCl₃, CF₂Cl and CHCl₂.

5. Process according to anyone of claims 1 to 4, wherein R³, R⁴ and R⁵ are H.

6. Process according to anyone of claims 1 to 5, which further comprises a step or reaction steps selected from the group consisting of d), e) and f):
d) reacting a compound of formula (VII) with a compound of formula (VIII) to obtain the compound of formula (IX), which is subsequently reacted with NH₃ or NH₄R⁷, wherein R⁷ is selected from the group consisting of OH, Br and Cl to obtain the compound of formula (II) 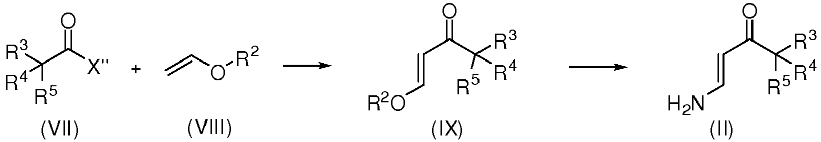
e) reacting a compound of formula (X) with a compound NH₃ or NH₄R⁷, wherein R⁷ is selected from the group consisting of OH, Br and Cl, to obtain the compound of formula (II), wherein M is an alkali metal cation, preferably sodium 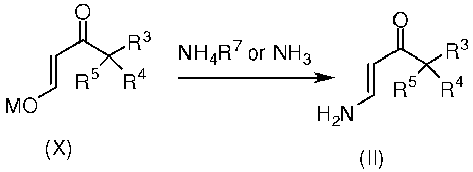
f) reacting a compound of formula (XI) with compound NH₃ or NH₄R⁷, wherein R⁷ is selected from the group consisting of OH, Br and Cl to obtain the compound of formula (II)

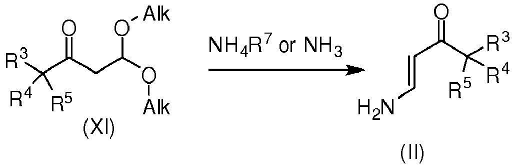

7. Process for the manufacture of a compound according to formula (XII), 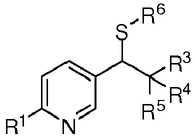 which comprises the process according to anyone of claims 1 to 6, and wherein R⁶ is selected from the group consisting of H and C₁-C₄-alkyl, wherein C₁-C₄-alkyl is optionally substituted.

8. Process according to claim 7, which comprises one of the steps selected from the group consisting of
g) Reduction of compound (III) to obtain compound (XIII), halogenation of compound (XIII) to obtain compound (XIV), wherein X' is selected from Br, Cl and I, and thioalkylation of compound (XIV) to obtain compound (XII) 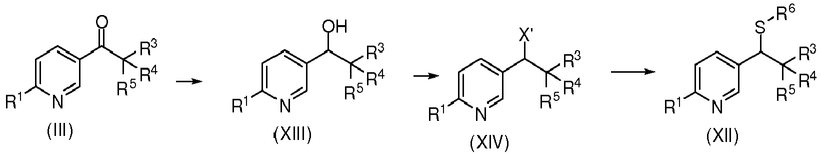
h) Dehydratization of (III), when at least R³ is H to obtain (XV); thioalkylation to obtain (XVI) and subsequent reduction to obtain (XII), wherein X" is selected from Br and Cl 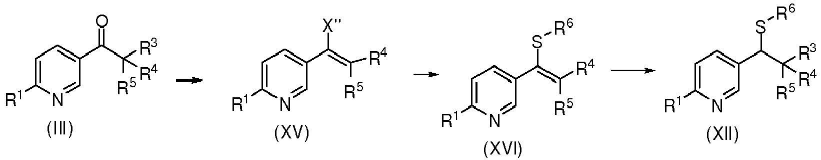
i)Thiation of (III) to obtain (XVII); reduction to obtain (XVIII) and subsequent alkylation to obtain (XII)

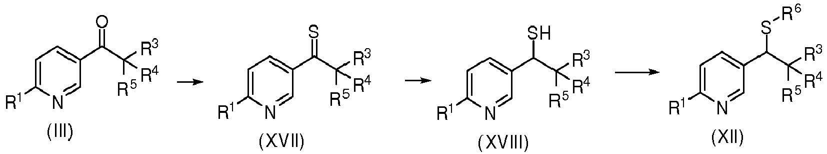

9. Process for the manufacture of an agriculturally or pharmaceutically active compound or an intermediary thereof, comprising the process according to anyone of claims 1 to 8.

10. Process according to claim 9, wherein the agriculturally active compound is a sulfoximines, in particular sulfoxaflor.

11. Use of the compound of formula (XII) obtained by the process according to anyone of claims 1 to 10 for the manufacture of an agriculturally or pharmaceutically active compound or an intermediary thereof, preferably wherein the agriculturally active compound is a sulfoximine, in particular sulfoxaflor.
